(19) Europäisches Patentamt
European Patent Office
Office européen des brevets

(11) **EP 0 783 038 B1**

(12) **FASCICULE DE BREVET EUROPEEN**

(45) Date de publication et mention
de la délivrance du brevet:
**04.04.2007 Bulletin 2007/14**

(51) Int Cl.:
*C12N 15/33* (2006.01)   *C12N 15/35* (2006.01)
*C12N 15/40* (2006.01)   *C07K 14/015* (2006.01)
*A61K 39/295* (2006.01)

(21) Numéro de dépôt: **96400009.5**

(22) Date de dépôt: **02.01.1996**

(54) **Pseudo-particules virales recombinantes et applications vaccinales et antitumorales**

Rekombinante virale Pseudopartikeln und deren Verwendung als Impfstoff oder als antitumorale Wirkstoff

Recombinant viral pseudoparticles and their applications as vaccines or as antitumour agents

(84) Etats contractants désignés:
ES FR

(43) Date de publication de la demande:
**09.07.1997 Bulletin 1997/28**

(73) Titulaires:
• **INSTITUT PASTEUR**
**75724 Paris Cédex 15 (FR)**
• **Immunologia Y Genetica Aplicada, S.A.**
**(Ingenasa)**
**28037 Madrid (ES)**

(72) Inventeurs:
• **Casal, Ignacio**
**E-22039 Madrid (ES)**
• **Sedlik, Christine**
**F-95100 Argenteuil (FR)**
• **Sarraseca, Javier**
**E-28041 Madrid (ES)**
• **Leclerc, Claude**
**F-75015 Paris (FR)**

(74) Mandataire: **Phélip, Bruno**
**c/o Cabinet Harlé & Phélip**
**7, rue de Madrid**
**75008 Paris (FR)**

(56) Documents cités:
**WO-A-91/04330**

• **JOURNAL OF GENERAL VIROLOGY, vol. 76, no. 9, Septembre 1995, READING GB, pages 2361-2368, XP002005309 C.SEDLIK ET AL.: "Immunogenicity of poliovirus B and T cell epitopes presented by hybrid porcine parvovirus particles"**

• **PROCEEDINGS OF THE NATIONAL ACADEMY OF SCIENCES OF USA, vol. 91, Août 1994, WASHINGTON US, pages 8507-8511, XP002005310 K.MIYAMURA ET AL.: "Parvovirus particles as platforms for protein presentation"**

• **JOURNAL OF VIROLOGY, vol. 69, no. 4, Avril 1995, pages 2187-2193, XP002005311 D.MOSKOPHIDIS ET R.M.ZINKERNAGEL: "Immunobiology of cytotoxic T- cell escape mutants of lymphocytic choriomeningitis virus"**

• **BROWN CAROLINE S. ET AL: 'Chimeric parvovirus B19 capsids for the presentation of foreign epitopes.' VIROLOGY vol. 198, 1994, pages 477 - 488**

• **SCHIRMBECK REINHOLD ET AL: 'Immunization with soluble hepatitis B virus surface protein elicits Immunization with soluble hepatitis B virus surface protein elicits murine H-2 class I- restricted CD8+ cytotoxic T lymphocyte responses in vivo.' JOURNAL OF IMMUNOLOGY vol. 152, 01 Février 1994, BALTIMORE, USA, pages 1110 - 1119**

• **LAYTON G.T. ET AL: 'Induction of HIV-specific cytotoxic T lymphocytes in vivo with hybrid HIV-1 V3:Ty-virus-like particles.' JOURNAL OF IMMUNOLOGY vol. 151, 15 Juillet 1993, BALTIMORE, USA, pages 1097 - 1107**

• **Calin-Laurens V. et al: 'Can one predict antigenic peptides for MHC class I-restricted cytotoxic T lymphocytes useful for vaccination?', Vaccine 11 (9): 974 - 978 (1993) Abstract only**

## Description

La présente invention concerne des pseudo-particules virales recombinantes d'un virus de la famille des *Parvoviridae*, utiles notamment pour induire des réponses lymphocytaires T cytotoxiques (CTL) in vivo à un niveau élevé.

Elle a encore pour objet l'utilisation de ces pseudo-particules pour la fabrication de vaccins ou de médicaments anti-tumoraux.

Elle a également trait à des compositions comprenant lesdites pseudo-particules dans un véhicule et / ou un diluant physiologiquement acceptable.

Selon Fernandez et al. (Médecine/Sciences, 1995, 11, 975-983), la réponse à médiation cellulaire, essentielle dans le cas d'une tumeur, peut être divisée en deux parties:

- d'une part la mise en route de la réponse qui fait intervenir des lymphocytes TCD4$^+$ et des cellules présentatrices de l'antigène (CPA), et
- d'autre part la réponse effectrice cytotoxique dans laquelle interviennent les lymphocytes TCD8$^+$.

Les lymphocytes TCD4$^+$ activés vont proliférer et sécréter des cytokines alors que les lymphocytes TCD8$^+$ activés vont proliférer et se différencier en lymphocytes T cytotoxiques (CTL).

Ces deux familles de lymphocytes ont donc des fonctions très différentes et sont activées par des mécanismes distincts. La stimulation des lymphocytes CD4$^+$ nécessite l'association d'un peptide issu de la dégradation de l'antigène à des molécules de classe II du Complexe Majeur d'Histocompatibilité (CMH), alors que celle des cellules CD8$^+$ nécessite l'association d'un peptide à des molécules de classe I du CMH.

On a déjà proposé un certain nombre de stratégies prophylactiques ou thérapeutiques pour lutter contre des maladies d'origines diverses chez l'homme et l'animal causées par des virus, des bactéries, des parasites ou contre des maladies cancéreuses ou tumorales.

On connaît, par exemple, l'induction de réponses CTL in vivo par des vecteurs " vivants ". Cependant l'innocuité de ces vecteurs n'est pas garantie, notamment chez des individus immunodéprimés. D'autre part, les systèmes impliquant la réplication de ces vecteurs " vivants " ne conservent pas leur efficacité chez des individus immuns.

Martinez et al., enseignent dans Vaccine (vol. 10, pp. 684-690, 1992) l'utilisation de capsides vides de parvovirus de porc (PPV) dans la vaccination des porcs. La famille des *Parvoviridae* regroupe des virus distribués très largement chez des mammifères tels que porcs, bovins, chats, lapins, rats et chez l'homme. Cependant, les parvovirus sont relativement spécifiques des mammifères hôtes. Le parvovirus de porc (PPV), en particulier, est responsable de nombreuses infections dans les élevages industriels de porcs. Le parvovirus de porc (PPV) est constitué d'une particule isométrique non enveloppée de 20 nm de diamètre et à symétrie icosahédrique, contenant une molécule d'ADN simple brin. Il est composé de deux protéines de capsides, VP1 (83 kDa) et VP2 (64 kDa), et d'une troisième protéine, VP3 résultant de la protéolyse de VP2.

La protéine VP2 peut être produite par des cellules d'insectes à l'aide d'un système de baculovirus recombinant et les protéines VP2 obtenues sont capables de s'auto-assembler pour former des pseudo-particules virales qui ont la même taille que celle du virion natif. La vaccination de porcs contre le PPV est réalisée en les immunisant avec un mélange desdites capsides avec l'association adjuvante Alhydrogel (à 50 %) + Quil A 500 µg (Superfos). Un enseignement comparable à celui de cet article est retrouvé dans les demandes EP-551.449 et EP-554.414 qui indiquent de plus que des épitopes correspondant à d'autres protéines virales peuvent être incorporés aux capsides, sans toutefois préciser la nature de ces épitopes.

La demande EP-647.655 est quant à elle relative à des peptides synthétiques correspondant à des sites antigéniques de VP2. Il ne s'agit donc pas de la protéine entière.

Sedlik et al. décrivent, dans Journal of General Virology (76 : 2361-2368, (1995)), l'utilisation de particules hybrides de PPV comme vecteurs de deux épitopes du virus de la polio, C3 : B et C3 : T reconnus respectivement par des lymphocytes B et TCD4$^+$. Les particules contenant l'épitope C3 : TCD4$^+$ ne se révèlent capables de stimuler les réponses prolifératives que de cellules T, CD4$^+$ et non de cellules T CD8$^+$. Outre cette spécificité de stimulation, les réponses restent d'un niveau peu élevé, insuffisant pour une immunisation efficace. En tout état de cause, l'induction de ces réponses requiert obligatoirement l'utilisation d'un adjuvant (hydroxyde d'aluminium ou adjuvant complet de Freund).

En outre, les essais de modification des pseudo-particules en vue d'incorporer des épitopes hétérologues se heurtent à des difficultés comme l'inhibition de la formation des pseudo-particules . Même lorsque ces dernières se forment, l'épitope hétérologue n'est pas nécessairement immunogène, l'épitope pouvant par exemple se situer dans un site où il est incapable de prendre sa conformation naturelle ou d'être produit par les cellules présentant l'antigène.

Par ailleurs à l'instar des vaccins à vecteurs "vivants", l'innocuité de ces particules hybrides associées aux adjuvants n'est pas totalement assurée, une toxicité pouvant provenir de certaines propriétés des adjuvants susceptibles d'induire des effets secondaires néfastes pour l'organisme.

Il ressort donc de l'état de la technique que l'on ne connaissait pas de système fiable, efficace permettant de stimuler la réponse lymphocytaire T-cytotoxique, sans risque pour la santé de l'individu traité et sans utilisation d'adjuvants immunostimulateurs.

Le demandeur a montré qu'il était possible d'induire spécifiquement une réponse lymphocytaire CD8+, c'est-à-dire une réponse cytotoxique, à l'aide de pseudo-particules portant des épitopes susceptibles de s'associer avec au moins une molécule de classe I du CMH.

La présente invention a pour objet des pseudo-particules virales recombinantes constituées par auto-assemblage de la protéine de structure virale VP2 de parvovirus de porc (PPV), ayant une taille comprise entre environ 20 et 60 nm et formant une structure approximativement isocaédrique, ladite protéine étant modifiée à l'extrémité N-terminale par la présence d'au moins un épitope comprenant une séquence de 8 ou 9 acides aminés susceptible de s'associer à une molécule de classe I du complexe majeur d'histocompatibilité (CMH), ladite association étant reconnue par des lymphocytes T CD8+ cytotoxyques.

L'association formée entre cette séquence et les molécules de classe I est reconnue par le récepteur de lymphocytes T CD8+ et induit une différenciation et une prolifération de ces lymphocytes.

Avantageusement, ladite séquence est bordée de régions dites flanquantes susceptibles de moduler sa dégradation, c'est-à-dire de faciliter sa production lors de la dégradation de l'antigène, ou de faciliter sa fixation aux molécules de classe I du CMH.

Les régions flanquantes peuvent correspondre aux acides aminés flanquant l'épitope, T, CD8+ dans son environnement naturel. De manière générale, elles peuvent être composées de plusieurs acides aminés, et en particulier d'alanine ou de lysine.

Les pseudo-particules conformes à l'invention présentent un premier intérêt, provenant de la sécurité de ce mode d'immunisation, le vecteur mis en oeuvre étant non-réplicatif, constitué d'un seul type de protéine virale et pouvant être administré même à des individus immunodéprimés.

Un second avantage des pseudo-particules selon l'invention réside dans l'absence de réactions croisées avec le parvovirus humain et donc dans l'absence de problèmes liés à une pré-immunisation des individus, aboutissant éventuellement à une faible immunogénicité de ce type de vaccins recombinants.

En outre les pseudo-particules selon l'invention ne nécessitent pas la réplication du vecteur et peuvent donc conserver leur efficacité chez des individus immuns.

Egalement, les pseudo-particules conformes à l'invention possèdent une excellente immunogénicité - une seule immunisation suffit - et peuvent donc être utilisées en l'absence d'adjuvant immunostimulateur.

L'épitope peut être constitué par toute séquence se fixant à une molécule du CMH de classe I et susceptible d'induire une cytotoxicité T lymphocytaire. Il peut être en particulier un épitope TCD8+ d'un virus, tel que le VIH-1 ou le VIH-2, ou le virus de la grippe, ou un épitope tumoral ou exprimé par des cellules cancéreuses.

Selon un mode avantageux de réalisation, les pseudo-particules selon l'invention sont caractérisées en ce que l'épitope sélectionné est l'épitope CTL CD8+ contenu dans la région 118-132 de la nucléoprotéine du virus de la chorioméningite lymphocytaire.

L'épitope peut néanmoins être tout autre épitope susceptible d'induire une réponse CTL, tel que par exemple l'un des épitopes suivants:

- Les épitopes des protéines Env gp120, Env gp41, Gag p17, Gag p24, Gag p15, Pol et Nef, des virus VIH et les épitopes des protéines Gag, et nef du virus VIS et Gag du virus VIH-2, tels que définis par VENET and WALKER (AIDS 1993, Vol.7, suppl. 1, 119-120).
- les épitopes de protéines exprimées par des tumeurs se développant chez l'être humain, telles que les protéines exprimées par les gènes MAGE-1, MAGE-3, BAGE, GAGE-1,2, HER-2/neu, et d'antigènes de différenciation mélanocytique, tels que la tyrosinase, Pmel17gp100, Melan-A$^{MART-1}$, et gp$^{75TRP1}$, définis par VAN DEN EYNDE et BRICHARD (Current Opinion in Immunology, 1975, 7, 674-681).

De manière avantageuse, lesdites pseudo-particules virales recombinantes sont susceptibles d'être obtenues par un procédé comprenant une étape d'expression dans le baculovirus, de la protéine chimérique constituée de la protéine VP2 du PPV et dudit épitope.

Les pseudo-particules conformes à l'invention sont obtenues, de façon encore plus préférée, par auto-assemblage de la protéine VP2 de PPV modifiée par la présence, à son extrémité N-terminale, d'au moins un épitope de la nucléoprotéine de LCMV.

La présente invention a aussi pour objet l'utilisation des pseudo-particules en quantité immunisante efficace, pour l'induction de réponses CTL in vivo à un niveau élevé.

Par l'expression " quantité immunisante efficace ", on entend une quantité choisie en fonction de la voie d'administration et du poids de l'individu de pseudo-particules selon l'invention. Avantageusement la quantité administrée sera comprise entre 10 et 500$\mu$g de pseudo-particules par individu.

La présente invention a en outre pour objet une composition comprenant les pseudo-particules dans un véhicule et/ou un diluant acceptables d'un point de vue immunologique. Comme diluant on peut utiliser une solution aqueuse tamponnée au voisinage de pH 7 (soluté physiologique).

De manière avantageuse, la composition conforme à la présente invention est exempte d'adjuvant immunostimulant. Elle peut néanmoins en contenir, si nécessaire, un tel adjuvant qui peut être de l'hydroxyde d'aluminium.

Les pseudo-particules selon la présente invention peuvent aussi être utilisées dans un procédé de stimulation in vitro des réponses T cytotoxiques de lymphocytes provenant de sujets atteints d'infections virales ou tumorales comprenant la mise en contact des lymphocytes des sujets avec les pseudo-particules. Dans ce cas, les cellules sont, après traitement, réadministrées aux patients, par exemple par injection.

La présente invention a encore pour objet l'utilisation des pseudo-particules conformes à l'invention dans la préparation d'un vaccin, de préférence à dose unique, notamment antitumoral ou antiviral. Il peut être administré par les voies habituelles pour les vaccins, à savoir par voie intramusculaire, intradermique, sous-cutanée ou éventuellement par voie orale ou toute autre voie permettant l'activation d'une réponse CTL.

L'invention sera illustrée sans être aucunement limitée par la description qui suit, faite en référence aux dessins annexés sur lesquels:

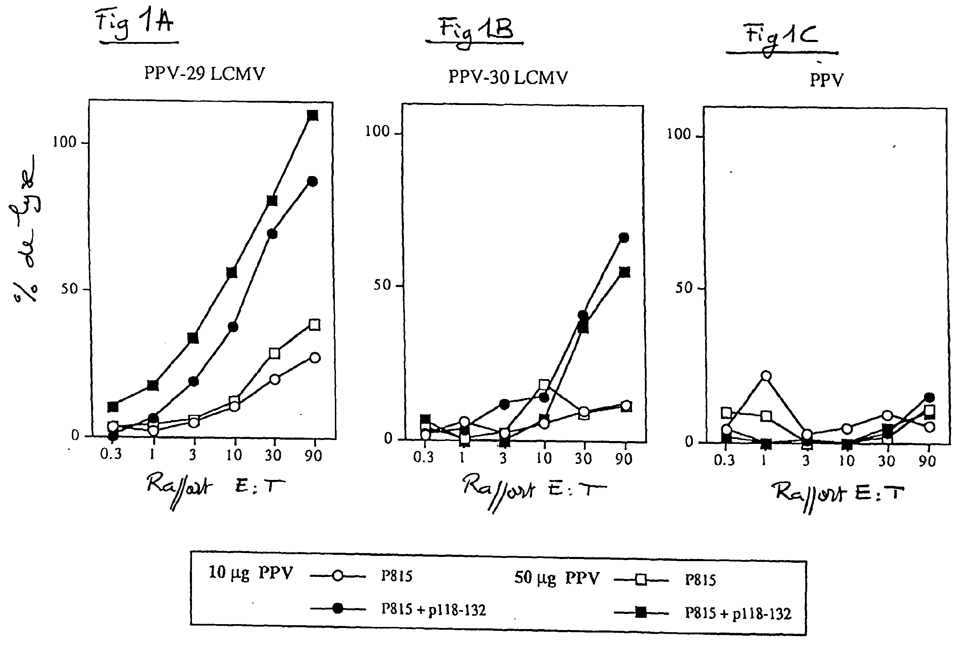

La Figure 1 comprenant trois graphiques 1 (a), 1(b) et 1(c) illustre l'immunisation de souris, à deux reprises, à j0 et j21 avec respectivement 10 et 50 $\mu$g de pseudo-particules conformes à l'invention, sans adjuvant. A j28, les splénocytes sont stimulés in vitro pendant 5 jours avec des splénocytes syngéniques pré-incubés avec le peptide synthétique 118-132. L'activité lymphocytaire T cytotoxique est mesurée sur des cibles P815 pré-incubées avec du milieu ou avec le peptide synthétique 118-132. Les résultats sont exprimés en % de lyse, le rapport E:T étant porté en abscisse, cette expression E/T désignant le rapport lymphocytes effecteurs/cellules cibles. La figure 1(a) concerne les résultats obtenus avec la particule PPV-29LCMV, la figure 1(b) les résultats obtenus avec la particule PPV-30 LCMV et la figure 1(c) à titre comparatif avec les pseudo-particules non modifiées.

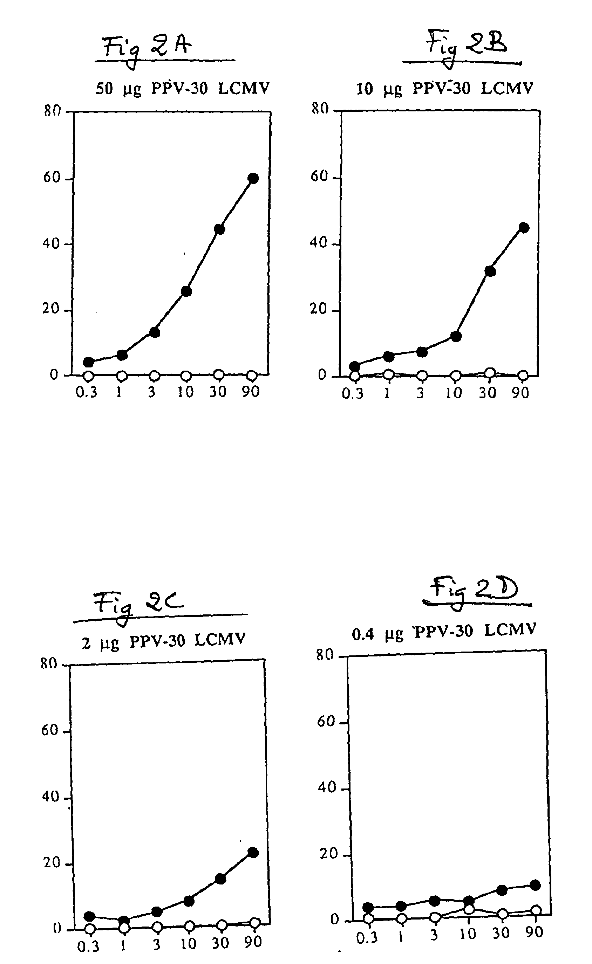

La figure 2 comprenant 7 graphiques 2(a), 2(b), 2(c), 2(d), 2(e), 2(f) et 2 (g) illustre l'immunisation de souris, à deux reprises, à j0 et j21, avec différentes doses respectivement de 50 $\mu$g pour la figure 2(a), 10 $\mu$g pour la figure 2(b), 2 $\mu$g pour la figure 2(c), 0,4 $\mu$g pour la figure 2(d), 0,08 $\mu$g pour la figure 2(e) de pseudo-particules selon l'invention, sans adjuvant. A j28, les splénocytes sont stimulés in vitro pendant 5 jours avec des splénocytes syngéniques pré-incubés avec le peptide synthétique 118-132. L'activité lymphocytaire T cytotoxique est mesurée sur des cibles P815 pré-incubées avec du milieu ou avec le peptide synthétique 118-132. Les souris témoins ont reçu une injection à j21 de 100 $\mu$g de peptide synthétique 118-126 en adjuvant incomplet de Freund (IFA) (fig. 2g) ou de pseudo-particules témoins (fig. 2f). Les résultats sont exprimés en % de lyse, de la même manière qu'à la figure 1.

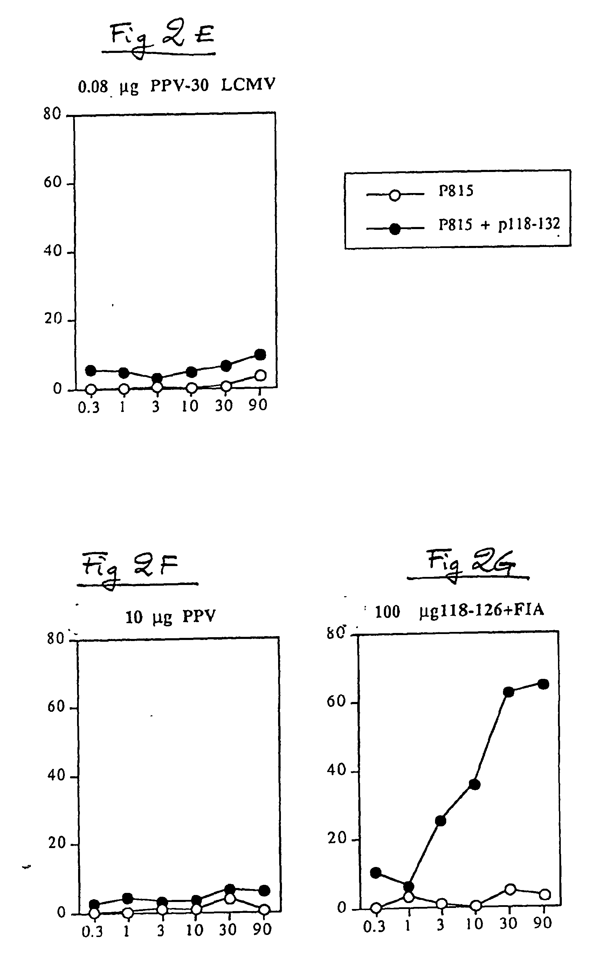

La figure 3 comprenant, d'une manière similaire à la Figure 1, trois graphiques 3(a), 3(b) et 3(c) illustre l'immunisation de souris par une seule injection de 10 $\mu$g ou de 50 $\mu$g de pseudo-particules conformes à l'invention, sans adjuvant. A j14, les splénocytes sont stimulés in vitro pendant 5 jours avec des splénocytes syngéniques pré-incubés avec le peptide synthétique 118-132. L'activité lymphocytaire T cytotoxique est mesurée sur des cibles P815 pré-incubées avec du milieu ou avec le peptide synthétique 118-132. Les résultats sont exprimés en % de lyse, de la même manière qu'à la Figure 1.

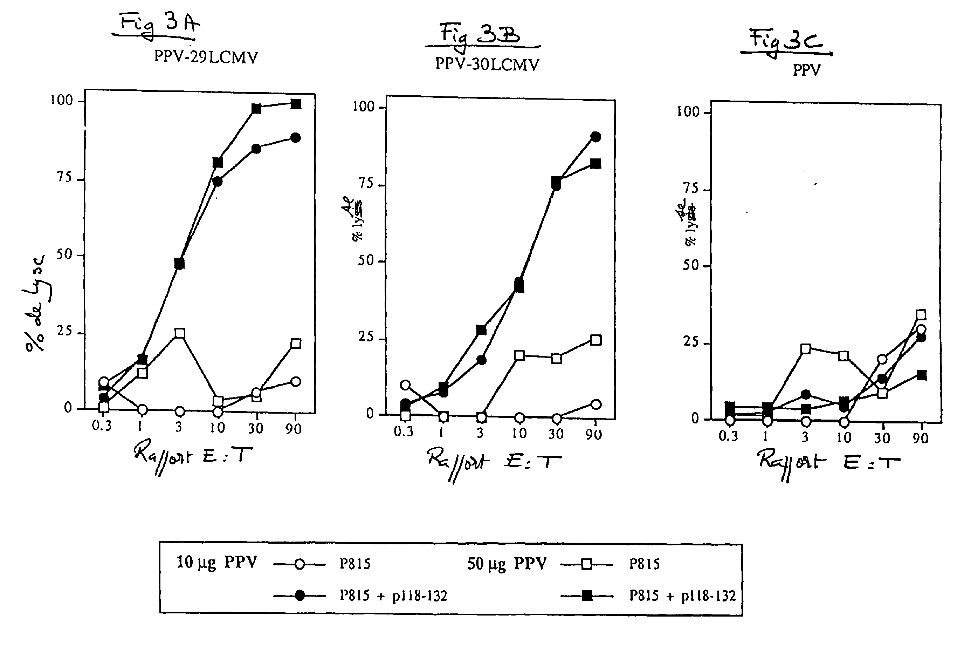

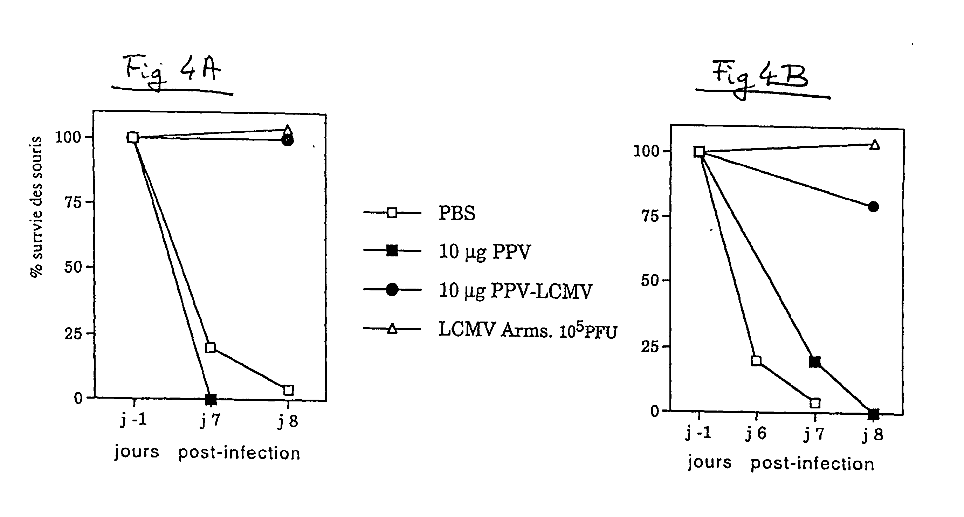

La figure 4 comprenant deux graphiques 4(a) et 4(b) illustre la protection de souris BALB/c, injectées par voie i.p. aux jours 0 et 21 avec du PBS, ou avec des pseudo-particules vides (PPV) ou exprimant la séquence 118-132 de la nucléoprotéine du LCMV (PPV-LCMV), ou avec le virus souche Armstrong (LCMV-Arms), contre l'infection par LCMV respectivement aux jours 28 (4a) et 70 (4b). Les souris ont été infectées par voie intracérébrale avec $10^{1,7}$.PFU/souris. La mortalité des souris est suivie quotidiennement pendant 10 jours et les résultats sont exprimés en % de survie parmi les animaux de chaque lot.

## EXEMPLE: INDUCTION DE LA CYTOTOXICITE T LYMPHOCYTAIRE PAR DES PSEUDO-PARTICULES SELON L'INVENTION.

L'épitope CTL hétérologue, CD8[+] contenu dans la région 118-132 de la nucléoprotéine de LCMV a été introduit dans le gène de VP2 du PPV sans altérer la formation ultérieure des pseudo-particules. Deux particules ont été obtenues, PPV-29LCMV et PPV-30LCMV, différant uniquement par un codon d'initiation. Ces protéines chimères ont été produites par un système de baculovirus et purifiées par précipitation au sulfate d'ammonium à partir du lysat de cellules d'insectes selon la méthode décrite dans Journal of General Virology 76 : pp. 2361-2368 (1995).

La capacité de ces particules recombinantes à induire des réponses lymphocytaires T cytotoxiques a été analysée in vivo chez la souris BALB/c.

**Matériel et méthodes**

**\* Insertion de l'épitope LCMV CD8± dans le site XhoI de l'extrémité N-terminale de VP2.**

Deux oligonucléotides codant pour l'épitope CD8+ et le codon d'initiation, et comprenant deux sites XhoI ont été synthétisés. Ils présentent les séquences suivantes:

```
5'TCGAGATGCGACCACAAGCTTCAGGAGTATACATGGGAAACCTAACAG
CACAAC3'
```

```
5'TCGAGTTGTGCTGTTAGGTTTCCCATGTATACTCCTGAAGCTTGTGGT
CGCATC3
```

Ces deux oligonucléotides complémentaires ont été fournis par MedProbe (Norvège).Ils ont été phosphorylés à l'aide de la polynucléotide kinase T4, annelés à 70˚C durant 15 minutes et ligaturés dans le site XhoI du plasmide pPPV29mod digéré par la XhoI ; qui contient le gène codant pour la PPV-VP2.

Des cellules d'*Escherichia coli* DH5 ont été transformées avec le mélange de ligature et étalées sur du milieu LB contenant 100 μg/ml d'ampicilline. Les recombinants contenant l'insert LCMV ont été sélectionnés, puis séquencés par le procédé à la didéoxy afin de déterminer l'orientation et l'intégrité des séquences insérées. Le clone recombinant contenant la séquence LCMV dans l'orientation adéquate a été appelé pPPV29mod/LCMV.

**\* Construction de vecteurs de transfert dans les baculovirus et sélection des baculovirus recombinants.**

La séquence VP2 chimérique a été obtenue à partir du vec- teur pPPV29mod/LCMV par digestion par BamHI et sous-clonée dans l'unique site de restriction BamHI du vecteur de transfert du baculovirus PacYM1.

Des clones recombinant ont été préparés comme décrit ci-dessus et analysés par la méthode de minipréparation d'ADN plasmidique et par cartographie de restriction. Les séquences d'insertion des clones positifs ont été à nouveau séquencées afin de vérifier à nouveau l'intégrité de l'épitope inséré ainsi que des séquences flanquantes. Finalement, l'ADN purifié par le phénol a été précipité par deux volumes d'éthanol. Le clone recombinant ainsi obtenu a été appelé pAcYM1/LCMV/ppv29mod.

Afin d'obtenir des baculovirus recombinants, un mélange de 2 μg d'ADN de vecteur de transfert purifié et de 500 ng d'ADN parental AcRP231acz+ a été ajouté à des cellules d'insectes Sf9 en présence du réactif de transfection DOTAP (Boehringer Mannheim), selon les instructions de cette Société. La transfection a été poursuivie jusqu'à ce que l'effet cytopathique soit total. Après 9 jours les cultures ont été récupérées et le surnageant clarifié a été utilisé pour l'isolement de plages de baculovirus recombinants selon les méthodes déjà connues de l'homme du métier et décrit par Sedlik et al. (J. Gen. Virol, 1995, 76, 2361-2368). Les clones recombinant ont été sélectionnés en utilisant leur phénotype blanc (plage blanche: recombinant; plage bleue: phénotype sauvage).

Les baculovirus recombinants ont été purifiés jusqu'à ce qu'aucune plage bleue ne puisse être détectée. Des stocks de virus présentant un titre important de baculovirus recombinant AcNPV.PPV-LCMV ($>10^8$) ont été préparés.

Un clone, appelé AcPP29-LMCV a été déposé le 20 Décembre 1995 sous le n˚V 95122022 auprès de l'European Collection of Animal Cell Cultures (ECACC).

**\* Analyse des protéines recombinantes.**

Les cellules Sf 9 ont été infectées avec les baculovirus recombinants à raison d'un taux d'infection de 1 pfu par cellule (une unité formant plage par cellule) et les extraits des cellules ont été collectés 72 heures après infection.

Du tampon de dissociation de protéine a été ajouté à chaque extrait cellulaire, et les mélanges ont été chauffés à 100˚C durant 5 minutes puis séparés sur SDS-9% PAGE.

Les gels ont été colorés à l'aide de bleu de Coomassie ou transférés sur des membranes de nitrocellulose à l'aide d'un équipement semi-sec, à 22 volts durant 30 minutes.

Les membranes ont été incubées avec du sérum de souris dirigé à l'encontre de l'épitope LCMV CD8+ (dilution 1/200) durant deux heures à température ambiante.

Après lavage les anticorps liés ont été détectés par la protéine A conjuguée à la péroxydase (dilution 1/2000) en utilisant du 4-chloro-naphtol comme substrat jusqu'à développement de la couleur. Les membranes ont ensuite été rincées avec de l'eau distillée afin d'arrêter la réaction. Après coloration des gels SDS par du bleu de Coomassie, une bande visible a 67 kD a été détectée dans les extraits de cellules Sf9 infectées avec les baculovirus recombinants. La taille observée correspond à la taille attendue pour la protéine chimérique VP2-LCMV. L'identité de cette protéine est confirmée par immunotransfert. L'antisérum de souris spécifique montré une réaction positive très claire avec la protéine de 67 kD des extraits cellulaires infectés. La localisation intracellulaire de la protéine recombinante a été analysée par immunotransfert de différentes fractions cellulaires. Ceci a permis de montrer que la protéine chimérique LCMV-VP2 est une protéine cytoplasmique soluble.

### * Purification des particules

Des cellules Sf9 ont été infectées à l'aide de baculovirus recombinant à un taux d'infection de 1 pfu par cellule. Les cellules ont été récoltées 72 heures après l'infection, lavées avec du PBS et lysées par choc osmotique à 4°C dans une solution de bicarbonate 25 mM. Les débris cellulaires ont été éliminés par centrifugation à basse vitesse. Les particules contenues dans les extraits ont été alors purifiées par précipitation par une solution saturée à 20% de sulfate d'ammonium. Le précipité est centrifugé à 15000 rpm durant 15 minutes, resuspendu dans du PBS et dialysé durant une nuit contre ce même tampon. L'identité et les propriétés des particules ont été confirmées par SDS-PAGE, immunotransfert et par microscopie électronique.

Ces particules purifiées ont été utilisées pour l'immunisation et l'induction de la cytotoxicité lymphocytaire (CTL).

### * Immunisation des souris.

Les souris reçoivent par voie intrapéritonéale une injection d'une composition contenant les pseudo-particules virales recombinantes conformes à l'invention (PPV-LCMV), ou vides (PPV), en l'absence d'adjuvant. Les témoins positifs sont des souris injectées, à j21, par voie sous-cutanée avec le peptide synthétique 118-126 (fourni par la société Neosystem, Strasbourg, France) en adjuvant incomplet de Freund (IFA). Des témoins négatifs reçoivent des pseudo-particules n'exprimant pas l'épitope LCMV.

### *Induction de cellules cytotoxiques

7 à 14 jours après la dernière immunisation, les rates sont prélevées sur les souris immunisées. $25 \times 10^6$ cellules immunes sont mises en culture, en présence de $25 \times 10^6$ splénocytes irradiés provenant de souris syngéniques naïves, en flacons contenant du milieu nutritif (RPMI 1640, 10% sérum de veau foetal, glutamine et antibiotique) pendant 5 jours à 37°C, 5% $CO_2$.

Les cellules stimulatrices sont sensibilisées in vitro avec le peptide synthétique 118-126 à à 0,05 $\mu$M ou avec le peptide synthétique 118-132 (également fourni par la société Neosystem, Strasbourg, France) à 0,5 $\mu$M.

### *Test de cytotoxicité

L'activité cytotoxique des lymphocytes effecteurs a été mesurée après 5 jours. Les cellules cibles P815 sont pré-incubées soit avec du milieu nutritif (témoin négatif), soit avec le peptide antigénique synthétique et marquées simultanément par le $^{51}$Cr pendant 1 h., à 37 °C. Ensuite, les lymphocytes effecteurs sont incubés avec les cellules marquées, à différents rapports effecteur / cible (rapport E / T) en milieu nutritif dans des plaques 96 puits à fond rond, pendant 4 à 5 h., à 37 °C. Les cibles sont incubées dans les mêmes conditions avec du milieu pour mesurer la libération spontanée du $^{51}$Cr et avec de l'acide chlorhydrique 1N pour mesurer la libération maximale de $^{51}$Cr. L'activité des lymphocytes cytotoxiques est déterminée par comptage de la radioactivité présente dans les surnageants de culture, correspondant au relargage du $^{51}$Cr par les cellules cibles lysées.

$$\% \text{ lyse spécifique} = \frac{\text{cpm expérimental} - \text{cpm spontané}}{\text{cpm maximal} - \text{cpm spontané}}$$

Ce calcul est réalisé avec les cellules cibles incubées dans du milieu nutritif (P815) et avec les cellules cibles incubées avec le peptide synthétique (P815 + p118-132), comme représenté par les courbes des Figures 1 à 3.

Résultats.

Comme on peut le constater d'après l'examen des Figures 1 à 3, une seule injection de 10 μg de pseudo-particules recombinantes présentant l'épitope T, CD8$^+$ du LCMV a permis d'atteindre pratiquement 100 % de lyse, c'est à dire autant que le témoin positif peptide synthétique p118-I26 + FIA, ce qui confirme que les pseudo-particules conformes à l'invention permettent l'induction de réponses CTL in vivo à un niveau élevé sans adjonction d'adjuvant.

Cette induction est spécifique de l'épitope inséré car aucune réponse T cytotoxique n'est obtenue lorsque les souris sont immunisées par des doses équivalentes de pseudo-particules n'exprimant pas l'épitope LCMV. Les cellules T cytotoxiques ainsi induites lysent spécifiquement les cellules cibles P815 recouvertes par le peptide correspondant à l'épitope inséré.

La figure 4 montre quant à elle que les souris auxquelles des pseudo-particules ont été injectées sont protégées contre l'infection virale à 28 jours, et que 4/5 souris sont protégées à 70 jours.

## Revendications

1. Pseudo-particules virales recombinantes **caractérisées en ce qu'**elles sont constituées par auto-assemblage de la protéine de structure virale VP2 de parvovirus de porc (PPV), **en ce qu'**elles ont une taille comprise entre 20 et 60 nm, et **en ce que** ladite protéine VP2 est modifiée à l'extrémité N-terminale par la présence d'au moins un épitope comprenant une séquence de 8 ou 9 acides aminés susceptible de s'associer à une molécule de classe I du complexe majeur d'histocompatibilité, ladite association étant reconnue par des lymphocytes T CD8[+] cytotoxiques.

2. Pseudo-particules selon la revendication 1 **caractérisées en ce que** ledit épitope est un épitope de la nucléoprotéine du virus de la chorioméningite lymphocytaire.

3. Pseudo-particules selon l'une des revendications 1 et 2 **caractérisées en ce que** ledit épitope est l'épitope CTL CD8[+] contenu dans la région 118-132 de la nucléoprotéine du virus de la chorioméningite lymphocytaire.

4. Pseudo-particules selon l'une quelconque des revendications 1 à 3 **caractérisées en ce qu'**elles sont susceptibles d'être obtenues par un procédé comprenant une étape d'expression dans le baculovirus de la protéine hybride formée de la protéine VP2 de parvovirus et dudit épitope.

5. Composition comprenant des pseudo-particules selon l'une quelconque des revendications 1 à 4, dans un véhicule et/ou un diluant physiologiquement acceptable.

6. Composition selon la revendication 5, **caractérisée en ce qu'**elle ne comprend pas d'adjuvant immunostimulateur.

7. Utilisation des pseudo-particules selon l'une quelconque des revendications 1 et 4, ou d'une composition selon la revendication 5 pour la fabrication de médicaments ou de vaccins pour l'induction de réponses CTL, *in vivo.*

8. Utilisation selon la revendication 7, pour la fabrication de vaccins antiviraux ou de médicaments antitumoraux.

9. Procédé de stimulation in vitro des réponses T cytotoxiques de lymphocytes provenant de sujets atteints d'infections virales ou tumorales comprenant la mise en contact des lymphocytes des sujets avec les pseudoparticules selon l'une des revendications 1 à 4.

## Claims

1. Recombinant viral pseudoparticles, **characterized in that** they are formed by self-assembly of at least the viral structural protein VP2 from porcine parvovirus (PPV), **in that** they have a size of between 20 and 60 nm, and **in that** the VP2 protein is modified, at its N-terminal end, by the presence of at least one epitope comprising a sequence of 8 or 9 amino acids, that may associate with a major histocompatibility complex class I molecule, said association being recognized by cytotoxic T CD8$^+$ lymphocytes.

2. Pseudoparticles according to claim 1, **characterized in that** said epitope is an epitope of the lymphocytic choriomeningitis virus nucleoprotein.

3. Pseudoparticles according to one of claims 1 and 2, **characterized in that** said epitope is CD8$^+$ CTL epitope contained in region 118-132 of the lymphocytic choriomeningitis virus nucleoprotein.

4. Pseudoparticles according to any one of claims 1 to 3, **characterized in that** they may be obtained by means of a method comprising a step consisting of expression, in the baculovirus, of the hybrid protein made up of the parvovirus VP2 protein and of said epitope.

5. Composition comprising pseudoparticles according to any one of claims 1 to 4, in a physiologically acceptable vehicle and/or diluent.

6. Composition according to claim 5, **characterized in that** it comprises no immunostimulatory adjuvant.

7. Use of the pseudoparticles according to any one of claims 1 to 4, or of a composition according to claim 5, for the manufacture of medicinal products or of vaccines for inducing CTL responses *in vivo.*

8. Use according to claim 7 for the manufacture of antiviral vaccines or of antitumour medicinal products.

9. Method of stimulating, *in vitro,* the cytotoxic T responses of lymphocytes originating from individuals affected with viral or tumoral infections, which comprises bringing the lymphocytes of the individuals into contact with the pseudoparticles according to one of claims 1 to 4.

**Patentansprüche**

1. Rekombinante Pseudoviruspartikel, **dadurch gekennzeichnet, dass** sie durch Selbstorganisation des viralen Strukturproteins VP2 des Schweineparvovirus (PPV) entstehen, dass sie eine Größe zwischen 20 und 60 nm haben und dass das Protein VP2 an seinem N-terminalen Ende durch die Anwesenheit wenigstens eines Epitops modifiziert ist, das eine Sequenz von 8 oder 9 Aminosäuren umfasst, die sich an ein Molekül der Klasse I des Haupthistokompatibilitätskomplexes assoziieren kann, wobei die Assoziation von cytotoxischen CD8-positiven T-Lymphocyten erkannt wird.

2. Pseudopartikel gemäß Anspruch 1, **dadurch gekennzeichnet, dass** das Epitop ein Epitop des Kernproteins des lymphozytären Choriomeningitis-Virus ist.

3. Pseudopartikel gemäß einem der Ansprüche 1 und 2, **dadurch gekennzeichnet, dass** das Epitop das CD8-positive CTL-Epitop ist, das im Bereich 118-132 des Kernproteins des lymphozytären Choriomeningitis-Virus enthalten ist.

4. Pseudopartikel gemäß einem der Ansprüche 1 bis 3, **dadurch gekennzeichnet, dass** sie durch ein Verfahren erhalten werden können, das einen Schritt der Expression des aus dem Protein VP2 von Parvovirus und dem Epitop gebildeten Hybridproteins in Baculovirus umfasst.

5. Zusammensetzung, die Pseudopartikel gemäß einem der Ansprüche 1 bis 4 in einem physiologisch annehmbaren Träger und/oder Verdünnungsmittel umfasst.

6. Zusammensetzung gemäß Anspruch 5, **dadurch gekennzeichnet, dass** sie kein immunstimulierendes Adjuvans umfasst.

7. Verwendung der Pseudopartikel gemäß einem der Ansprüche 1 und 4 oder einer Zusammensetzung gemäß Anspruch 5 zur Herstellung von Medikamenten oder Impfstoffen zur Induktion von CTL-Antworten in vivo.

8. Verwendung gemäß Anspruch 7 zur Herstellung von antiviralen Impfstoffen oder Antitumormedikamenten.

9. Verfahren zur in-vitro-Stimulation der cytotoxischen T-Antworten von Lymphocyten, die von Patienten stammen, die eine virale oder tumorale Infektion erlitten haben, umfassend das In-Kontakt-Bringen der Lymphocyten der Patienten mit den Pseudopartikeln gemäß einem der Ansprüche 1 bis 4.

Fig 1A

PPV-29 LCMV

Fig 1B

PPV-30 LCMV

Fig 1C

PPV

% de Lyse

Rapport E:T

Rapport E:T

Rapport E:T

| 10 μg PPV | —○— | P815 | 50 μg PPV | —□— | P815 |
| | —●— | P815 + p118-132 | | —■— | P815 + p118-132 |

EP 0 783 038 B1

## Fig 2A

### 50 µg PPV-30 LCMV

## Fig 2B

### 10 µg PPV-30 LCMV

## Fig 2C

### 2 µg PPV-30 LCMV

## Fig 2D

### 0.4 µg PPV-30 LCMV

Fig 2 E

0.08 µg PPV-30 LCMV

P815

P815 + p118-132

Fig 2 F

10 µg PPV

Fig 2G

100 µg118-126+FIA

Fig 3A
PPV-29LCMV

Fig 3B
PPV-30LCMV

Fig 3C
PPV

10 μg PPV —○— P815       50 μg PPV —□— P815

—●— P815 + p118-132       —■— P815 + p118-132

EP 0 783 038 B1

Fig 4A

% survie des souris

PBS
10 µg PPV
10 µg PPV-LCMV
LCMV Arms. $10^5$PFU

j -1    j 7    j 8

jours   post-infection

Fig 4B

j -1   j 6   j 7   j 8

jours   post-infection

EP 0 783 038 B1

LISTE DE SEQUENCES

    (1) INFORMATIONS GENERALES:

        (i) DEPOSANT:

            (A) NOM: INSTITUT PASTEUR
            (B) RUE: 25-28, rue du Docteur Roux
            (C) VILLE: PARIS
            (E) PAYS: FRANCE
            (F) CODE POSTAL: 75724 PARIS
            (G) TELEPHONE: 45 68 80 93
            (H) TELECOPIE: 250 609
            (I) TELEX: 250 609

            (A) NOM; IMMUNOLOGIA Y GENETICA APLICADA, S.A.
            (B) RUE: Hermanos Garcia Noblejas 4120
            (C) VILLE: MADRID
            (E) PAYS: ESPAGNE
            (F) CODE POSTAL: E-28037

        (ii) TITRE DE L'INVENTION: PSEUDO-PARTICULES VIRALES RECOMBINANTES ET APPLICATIONS VACCINALES ET ANTITUMORALES

        (iii) NOMBRE DE SEQUENCES: 2

        (iv) FORME DECHIFFRABLE PAR ORDINATEUR:

            (A) TYPE DE SUPPORT: Floppy disk
            (B) ORDINATEUR: IBM PC compatible
            (C) SYSTEME D' EXPLOITATION: PC-DOS/MS-DOS
            (D) LOGICIEL: Patent In Release #1.0, Version #1.30 (OEB)

    (2) INFORMATIONS POUR LA SEQ ID NO : 1:

        (i) CARACTERISTIQUES DE LA SEQUENCE:

            (A) LONGUEUR: 54 paires de bases
            (B) TYPE : nucléotide
            (C) NOMBRE DE BRINS: simple
            (D) CONFIGURATION : linéaire

        (ii) TYPE DE MOLECULE : ADN (génomique)

(iii) HYPOTHETIQUE: NON
(xi) DESCRIPTION DE LA SEQUENCE: SEQ ID NO: 1:
TCGAGATGCG ACCACAAGCT TCAGGAGTAT ACATGGGAAA CCTAACAGCA CAAC          54

(2) INFORMATIONS POUR LA SEQ ID NO: 2:

    (i) CARACTERISTIQUES DE LA SEQUENCE:

        (A) LONGUEUR: 54 paires de bases
        (B) TYPE: nucléotide
        (C) NOMBRE DE BRINS: simple
        (D) CONFIGURATION: linéaire

    (ii) TYPE DE MOLECULE: ADN (génomique)
    (iii) HYPOTHETIQUE: NON

    (xi) DESCRIPTION DE LA SEQUENCE: SEQ ID NO: 2:
TCGAGTTGTG CTGTTAGGTT TCCCATGTAT ACTCCTGAAG CTTGTGGTCG CATC          54